# EUROPEAN PATENT SPECIFICATION

(11) **EP 2 136 703 B1**
(45) Date of publication and mention of the grant of the patent: **28.03.2012**
(21) Application number: 08725598.0
(22) Date of filing: 14.02.2008
(51) Int. Cl.: A61B 5/0452, G06F 17/00, A61N 1/37, A61B 5/0464

(54) **SYSTEMS AND METHODS FOR ENHANCING CARDIAC SIGNAL FEATURES USED IN MORPHOLOGY DISCRIMINATION**
SYSTEME UND VERFAHREN ZUR VERBESSERUNG VON HERZSIGNAL-MERKMALEN IN DER MORPHOLOGISCHEN DISKRIMINIERUNG
SYSTEMES ET PROCEDES DE STIMULATION DES CARACTERISTIQUES DES SIGNAUX CARDIAQUES UTILISES DANS LA DISCRIMINATION MORPHOLOGIQUE

(30) Priority: 13.03.2007 US 717482
(43) Date of publication of application: 30.12.2009
(73) Proprietor: Cardiac Pacemakers, Inc., St. Paul MN 55112 (US)
(72) Inventor: DONG, Yanting, Shoreview, MN 55126 (US); MANIAK, Jeremy, Columbia Heights, MN 55421 (US); MEYER, Scott, Lakeville, MN 55044 (US); STALSBERG, Kevin, White Bear Lake, MN 55110 (US); SATHAYE, Alok, Boston, MA 02108 (US); ETTORI, Benjamin, Minneapolis, MN 55408 (US)
(74) Representative: Charig, Raymond Julian
(86) International application number: PCT/US2008/001986
(87) International publication number: WO 2008/112060

(56) References cited:
- EP-A- 1 050 271
- WO-A-2004/012596
- WO-A-2005/002669
- US-A- 5 817 027
- US-A1- 2003 083 713

## Description

### FIELD OF THE INVENTION

The present invention relates generally to medical devices and, more particularly, to cardiac devices and methods used for classification of cardiac events based on morphological analysis of cardiac signals.

### BACKGROUND OF THE INVENTION

When functioning normally, the heart produces rhythmic contractions and is capable of pumping blood throughout the body. However, due to disease or injury, the heart rhythm may become irregular resulting in diminished pumping efficiency. Arrhythmia is a general term used to describe heart rhythm irregularities arising from a variety of physical conditions and disease processes. Cardiac rhythm management systems, such as implantable pacemakers and cardiac defibrillators, have been used as an effective treatment for patients with serious arrhythmias. These systems typically include circuitry to sense electrical signals from the heart and a pulse generator for delivering electrical stimulation pulses to the heart. Leads extending into the patient's heart are connected to electrodes that contact the myocardium for sensing the heart's electrical signals and for delivering stimulation pulses to the heart in accordance with various therapies for treating the arrhythmias.

Cardiac rhythm management systems may pace the heart by stimulating the heart tissue to produce a contraction of the tissue. Pacemakers deliver a series of low energy pace pulses timed to assist the heart in producing a contractile rhythm that maintains cardiac pumping efficiency. Detecting if a pacing pulse "captures" the heart and produces a contraction allows the cardiac rhythm management system to adjust the energy level of pace pulses to correspond to the optimum energy expenditure that reliably produces capture. A pace pulse must exceed a minimum energy value, or capture threshold, to produce a contraction. It is desirable for a pace pulse to have sufficient energy to stimulate capture of the heart without expending energy significantly in excess of the capture threshold.

Cardiac rhythm management systems may also detect and terminate abnormal tachyarrhythmias using a variety of tiered therapies. These tiered therapies range from the delivery of anti-tachyarrhythmia pacing (ATP) to high energy shocks to terminate tachyarrhythmia or fibrillation. To effectively deliver these treatments, the CRM device must first identify the type of arrhythmia that is occurring.

Detecting various cardiac events, such as capture or a tachyarrhythmia event, may be accomplished by analyzing the morphology of a cardiac signal, such as an electrogram (EGM) or electrocardiogram (ECG). The morphological analysis may involve determining the presence and/or location of cardiac signal features in the cardiac signal. This type of analysis is simplified when the cardiac signal features used to detect the cardiac events are relatively consistent and can be discriminated from other signal features and from noise.

WO-A-2005/002669 relates to morphological analysis of cardiac events for estimating a frequency of a sampled cardiac rhythm signal and classifying the rhythm. The received signal is sampled and transformed into a curvature series. A lobe in the curvature series corresponds to a characteristic point in the sampled series. Characteristic points are selected based on a time of a lobe in the curvature series and, in one embodiment, an amplitude of the signal at the time of the lobe. A frequency of the sampled series is estimated by autocorrelating a function of the series of the characteristic points. In one embodiment, the function is a time difference function. The rhythm is classified by plotting the timewise proximity of characteristic points derived from an atrial signal with characteristic points derived from a ventricular signal. Regions of the plot are associated with a particular rhythm and the grouping of the data corresponds to the classification.

There is a need for methods and systems that enhance detection of cardiac signal features used to classify various cardiac events. The present invention fulfills these and other needs.

### SUMMARY OF THE INVENTION

The present invention is directed to methods and devices used to classify cardiac events based on morphological analysis of sensed signals. One embodiment of the invention involves a method for classifying a cardiac event as defined in claim 1. The method includes sensing a signal comprising a cardiac signal component and a noise signal component. The sensed signal is processed to alter morphology of the cardiac signal component. The altered morphology of the cardiac signal component enhances detection of one or more features of the cardiac signal component. The one of more features of the cardiac signal component are detected and the cardiac event is classified using the detected features.

In certain implementations, processing the sensed signal involves processing the sensed signal using adaptable signal processing parameters. The signal processing parameters may be automatically determined or may be manually determined by a physician. For example, the signal processing parameters may be selected to accentuate one or more desirable features of the cardiac signal component .

According to one aspect, the signal processing parameters may be determined to enhance a correlation coefficient between beats from cardiac episodes and a template.

Another preferred aspect of the invention involves detecting one or more undesirable features of the cardiac signal component and determining signal processing parameters that mitigate the undesirable features of the cardiac signal component. For example, the undesirable features may be detected using a gradient analysis or using a curvature analysis.

The undesirable features may be detected and/or the signal processing parameters enhanced based on signals produced using a pacing protocol (e.g., AAI pacing to simulate a tachyarrhythmia episode) and/or may be based on stored cardiac signals.

According to various applications, the cardiac events classified using the altered cardiac signal component may include cardiac pacing responses and/or tachyarrhythmia episodes.

Another embodiment of the invention is directed to a medical system used to classify cardiac events as defined in claim 6. The system includes sensing circuitry configured to sense a signal comprising a cardiac signal component and a noise component. Signal processing circuitry is configured to process the sensed signal to alter morphology of the cardiac signal component of the sensed signal. The altered cardiac signal component enhances detection of one or more features of the cardiac signal component. A feature detector is configured to detect the one or more features of the cardiac signal component. A cardiac event processor configured to classify a cardiac event using the detected features.

A filter may be used to alter the cardiac signal component. In one example, the filter includes a high pass corner frequency of about 3 Hz and a low pass corner frequency of about 100 Hz. The system may additionally include a noise filter configured to filter the sensed signal to reduce the noise component of the sensed signal.

Parameters of the signal processing circuitry may be derived from population data or may be based on individual characteristics of a patient. The parameters of the signal processing circuitry can be automatically adapted by the device or programmed by a physician.

According to one implementation, the signal processing circuitry comprises a bank of signal processing units. Each of the signal processing units may have associated signal processing parameters and/or may be optimized to recognize or classify a particular type cardiac event.

The above summary of the present invention is not intended to describe each embodiment or every implementation of the present invention. Advantages and attainments, together with a more complete understanding of the invention, will become apparent and appreciated by referring to the following detailed description and claims taken in conjunction with the accompanying drawings.

### BRIEF DESCRIPTION OF THE DRAWINGS

Figure 1 is a flow graph of a method for identification of cardiac events using signal processing for enhanced feature detection in accordance with embodiments of the invention;
Figures 2A and 2B are graphs that illustrate modifying the cardiac signal component of a sensed signal to enhance feature detection in accordance with embodiments of the invention;
Figures 3A-3D illustrate the improvement in accurate identification of a captured response to cardiac pacing when the cardiac signal component of the sensed signal is altered for enhanced feature detection in accordance with embodiments of the invention;
Figure 4 shows an implantable cardiac rhythm management (CRM) device that may be used to implement signal processing to enhance feature detection in accordance with the present invention;
Figure 5A presents a functional block diagram of CRM circuitry configured to implement signal processing to enhance feature detection and cardiac event identification in accordance with embodiments of the invention;
Figure 5B is a block diagram illustrating a bank of signal processing units where each signal processing unit is used to enhance cardiac signal features used in identification of a particular type of cardiac event in accordance with embodiments of the invention;
Figure 6 is a flow graph that illustrates a method for automatically determining signal processing parameters for feature enhancement in accordance with embodiments of the invention;
Figure 7A is a graph of a cardiac signal prior to processing, where the cardiac signal has a morphology exhibiting double positive peaks;
Figure 7B is a graph of the gradient of the cardiac signal of Figure 7A including zero crossings indicating undesirable multiple peaks;
Figure 7C is a graph of the cardiac signal after processing illustrating the reduction of the double peak morphology of Figure 7A;
Figure 7D is a graph of the gradient of the cardiac signal of Figure 7C illustrating a single zero crossing corresponding to the single peak of the cardiac signal;
Figure 8 is a graph of a cardiac signal illustrating feature points extracted for cardiac rhythm identification; and
Figure 9 illustrates windows drawn around feature point locations 1-8. These windows may be used to detect significant changes in curvature and/or the presence of multiple inflection points within the window area in accordance with embodiments of the invention.

While the invention is amenable to various modifications and alternative forms, specifics thereof have been shown by way of example in the drawings and will be described in detail below. It is to be understood, however, that the intention is not to limit the invention to the particular embodiments described. On the contrary, the invention is intended to cover all modifications, equivalents, and alternatives falling within the scope of the invention as defined by the appended claims.

### DETAILED DESCRIPTION OF VARIOUS EMBODIMENTS

In the following description of the illustrated embodiments, references are made to the accompanying drawings forming a part hereof, and in which are shown by way of illustration, various embodiments by which the invention may be practiced. It is to be understood that other embodiments may be utilized, and structural and functional changes may be made without departing from the scope of the present invention.

Detection of various cardiac events and/or conditions, including cardiac pacing response events and/or tachyarrhythmia events is accomplished through morphological analysis of the cardiac signal produced by the heart. The cardiac signal may be acquired via implanted electrodes coupled to a pacemaker, defibrillator, or other type of cardiac rhythm management system. Various types of cardiac events are associated with consistently occurring cardiac signal features that can be used to classify the type of cardiac event.

Detection of cardiac signal features used to classify cardiac events may be made more difficult due to morphological characteristics of the cardiac signal. For example, undesirable cardiac signal features that occur close in time to a feature used for event identification are problematic. These undesirable features cause variation in discriminating the feature used for event identification. For example, if cardiac event identification is based on the amplitude and timing of a cardiac signal peak, a cardiac signal exhibiting a single sharp peak is desirable. The presence of a double peak or a flattened peak may lead to variation in determining the peak coordinates and corresponding inaccuracy in event identification.

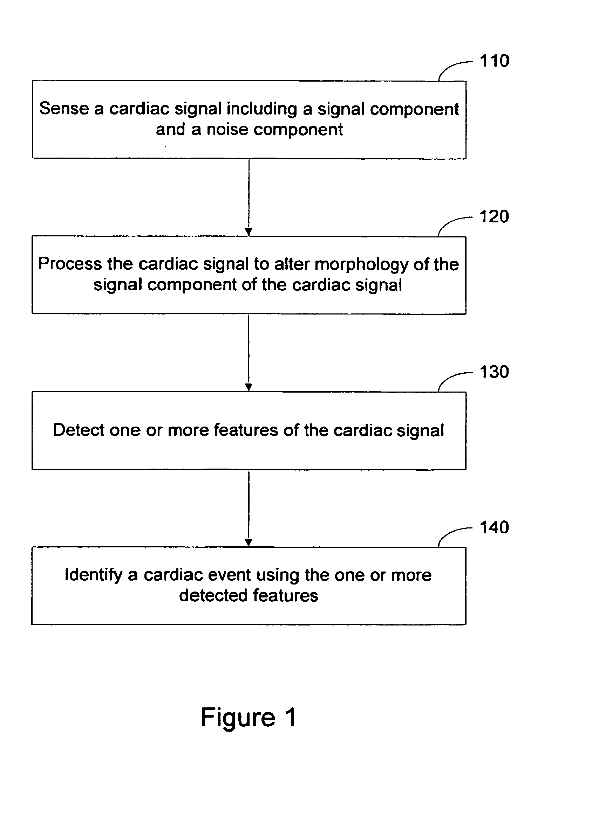

The flow graph of Figure 1 illustrates a method involving cardiac event identification using signal processing for enhanced feature detection in accordance with some embodiments. The method involves sensing 110 a signal that includes a cardiac signal component and a noise signal component. The sensed signal may derive from an electrical source, such as cardiac electrical activity, or a non-electrical source, such as a pressure signal, a respiration signal, or heart sound signal. The sensed signal is processed 120 to alter the morphology of the cardiac signal component. Alteration of the cardiac signal component involves signal processing to accentuate one or more features of the cardiac signal component alteration of the cardiac signal component does not necessarily modify the noise component. In some implementations, the noise component is filtered in a separate signal processing step. After alteration of the cardiac signal component, one or more features are detected 130 in the cardiac signal component. A cardiac event is identified 140 using the detected features.

A signal sensed via implantable or patient-external sensors, such as cardiac electrodes, pressure sensors, respiration sensors, accelerometers, or other sensors, generally includes a noise component superimposed on the cardiac signal component. For example, if the sensed signal is related to cardiac activity, the resultant composite signal is thus not necessarily an accurate representation of cardiac activity because the noise signal component is also present in the composite signal. For purposes of this discussion, noise signals are signals from sources other than the cardiac activity of the heart. Noise sources may include environmental noise, such as 60 Hz power line noise, myopotentials from skeletal muscle, motion artifacts, and/or other noise sources. Much effort has previously been directed to removing noise from the cardiac signal to achieve the "pure" cardiac signal component without noise or with a reduced level of noise. Reduction of noise is one technique used to facilitate morphological analysis of sensed signals to classify cardiac events. For example, increasing the signal to noise ratio of the sensed signal by filtering the noise component can enhance feature detection.

Noise reduction approaches attempt to reveal the cardiac signal from a signal that combines the cardiac signal with signal components from unwanted sources. The approaches of the present invention are directed to modifying the cardiac signal component of the sensed signal to enhance signal feature detection rather than to achieve the reduction of noise in the sensed signal. Modification of the cardiac signal component as illustrated by various embodiments herein may be accomplished with or without the process of noise reduction. For example, the signal processing techniques of the present invention may be applied to change the shape of the cardiac signal component to enhance detection of signal features present in the cardiac signal component of a sensed signal.

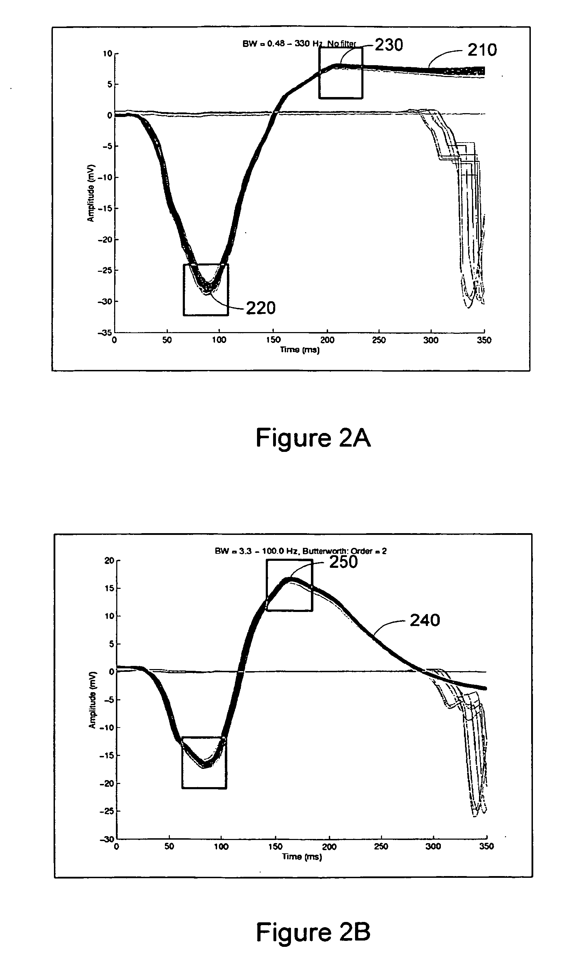

Signal processing for feature enhancement of the cardiac signal in accordance with embodiments of the invention may be achieved by processing the signal sensed via cardiac electrodes using one or more signal processors. The graphs of Figures 2A and 2B illustrate modification of the cardiac signal component to enhance feature detection. Figure 2A illustrates a cardiac signal 210 exhibiting a relatively sharp negative peak 220 and a flattened positive peak 230. Figure 2B illustrates the modified cardiac signal 240 after processing the signal to sharpen the positive peak 250.

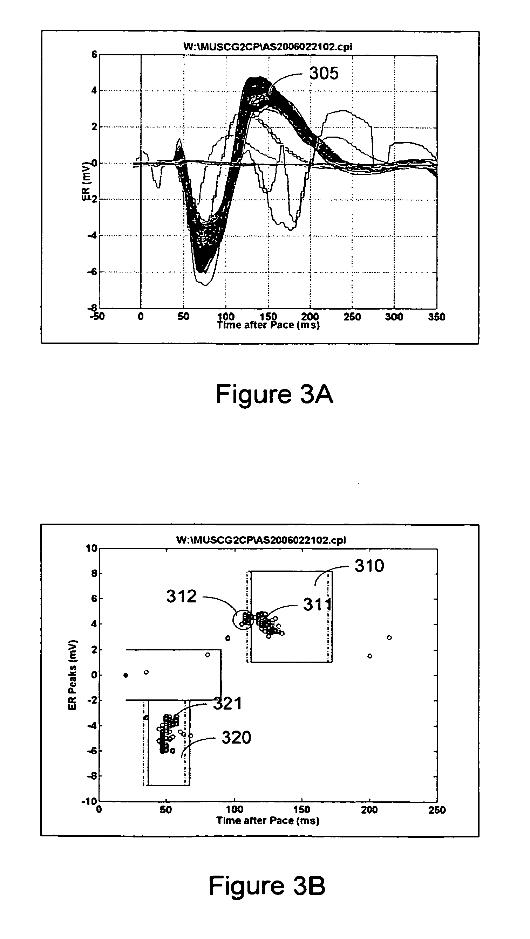

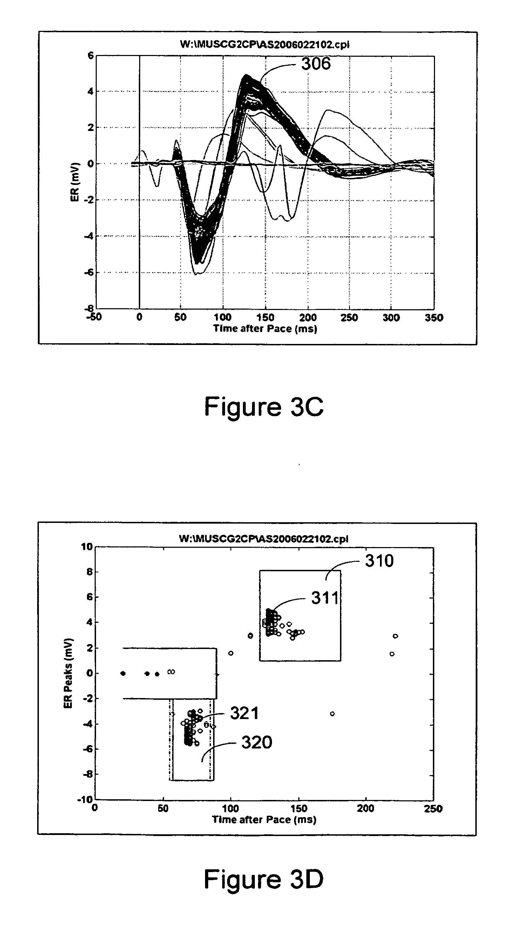

Signals processed to enhance feature detection lead to more accurate identification of cardiac events. Figures 3A-3D illustrate the improvement in accurate identification of a captured response to cardiac pacing. Figures 3A and 3C illustrate a number of cardiac signals 305, 306 sensed following pacing. A captured response is identified by negative and positive signal peaks that occur within detection windows 310, 320. Circles 311, 321 of Figures 3B and 3D indicate the location of the cardiac signal peaks of the various signals 305, 306. As can be seen in Figure 3A, the unprocessed signals 305 initially exhibit positive peaks having a somewhat flattened or double peak morphology. This morphology leads to group of signals having positive peaks 312 that fall outside the capture detection window 310. The signals having peaks 312 falling outside the capture detection window 310 would be incorrectly identified as a response other than a captured response.

After processing, the cardiac signals 306 exhibit altered morphology that enhances positive peak detection, as illustrated in Figures 3C and 3D. The signals 306 illustrated in Figure 3C have been processed, e.g., filtered, to alter the morphology of the signals 306. Figure 3D shows that the locations of the positive peaks 311 now fall within the capture detection window 310 resulting in accurate capture detection.

A system capable of processing signals to enhance feature detection may be implemented in a cardiac rhythm management (CRM) device such as an implantable cardiac defibrillator, pacemaker or resynchronization device. Although the present system is described in conjunction with device having a microprocessor-based architecture, it will be understood that the CRM device may be implemented in any logic-based integrated circuit architecture, if desired. Furthermore, methods of the present invention may be implemented in a patient-external device or a system that incorporates both patient-external and implantable components.

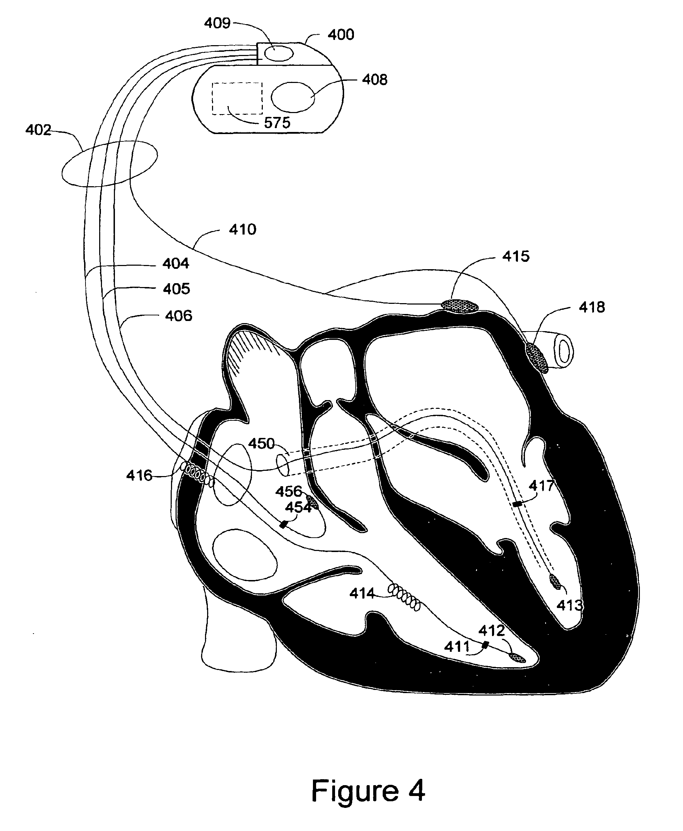

Referring now to Figure 4 of the drawings, there is shown a CRM device that may be used to implement signal processing to enhance feature detection in accordance with the present invention. The cardiac rhythm management system in Figure 4 includes a CRM device 400 electrically and physically coupled to a lead system 402. The housing and/or header of the CRM device 400 may incorporate one or more electrodes 408, 409 used to provide electrical stimulation energy to the heart and to sense cardiac electrical activity. The CRM device 400 may utilize all or a portion of the housing as a can electrode 409. The device 400 may include an indifferent electrode 408 positioned, for example, on the header or the housing of the device 400. If the device 400 includes both a can electrode 409 and an indifferent electrode 408, the electrodes 408, 409 typically are electrically isolated from each other.

The lead system 402 includes implantable electrodes used to detect electric cardiac signals produced by the heart and to provide electrical energy to the heart under certain predetermined conditions to treat cardiac arrhythmias. The lead system 402 may include one or more electrodes used for pacing, sensing, and/or defibrillation. In the embodiment shown in Figure 4, the lead system 402 includes an intracardiac right ventricular (RV) lead system 404, an intracardiac right atrial (RA) lead system 405, an intracardiac left ventricular (LV) lead system 406, and an extracardiac left atrial (LA) lead system 410. The lead system 402 of Figure 4 illustrates one embodiment that may be used in connection with the signal feature enhancement methodologies described herein. Other leads and/or electrodes may additionally or alternatively be used.

The lead system 402 may include intracardiac leads 404, 405, 406 inserted into a patient's heart and electrically coupled to the cardiac myocardium. The intracardiac leads 404, 405, 406 include various electrodes positionable within the heart for sensing electrical activity of the heart and for delivering electrical stimulation energy to the heart, for example, pacing pulses and/or defibrillation shocks to treat various arrhythmias of the heart.

As illustrated in Figure 4, the lead system 402 may include one or more extracardiac leads 410 having electrodes, e.g., epicardial electrodes, positioned at locations outside the heart for sensing and pacing one or more heart chambers.

The right ventricular lead system 404 illustrated in Figure 4 includes an SVC-coil 416, an RV-coil 414, an RV-ring electrode 411, and an RV-tip electrode 412. The right ventricular lead system 404 extends through the right atrium 420 and into the right ventricle 419. In particular, the RV-tip electrode 412, RV-ring electrode 411, and RV-coil electrode 414 are positioned at appropriate locations within the right ventricle for sensing and delivering electrical stimulation pulses to the heart. The SVC-coil 416 is positioned at an appropriate location within the right atrium chamber of the heart or a major vein leading to the right atrial chamber of the heart.

In one configuration, the RV-tip electrode 412 referenced to the can electrode 409 may be used to implement unipolar pacing and/or sensing in the right ventricle 419. Bipolar pacing and/or sensing in the right ventricle may be implemented using the RV-tip 412 and RV-ring 411 electrodes. In yet another configuration, the RV-ring 411 electrode may optionally be omitted, and bipolar pacing and/or sensing may be accomplished using the RV-tip electrode 412 and the RV-coil 414, for example. The RV-coil 414 and the SVC-coil 416 are defibrillation electrodes.

The left ventricular lead 406 includes an LV distal electrode 413 and an LV proximal electrode 417 located at appropriate locations in or about the left ventricle and used for pacing and/or sensing the electrical signals of the left ventricle. The left ventricular lead 406 may be guided into the right atrium of the heart via the superior vena cava. From the right atrium, the left ventricular lead 406 may be deployed into the coronary sinus ostium, the opening of the coronary sinus 450. The lead 406 may be guided through the coronary sinus 450 to a coronary vein of the left ventricle. This vein is used as an access pathway for leads to reach the surfaces of the left ventricle which are not directly accessible from the right side of the heart. Lead placement for the left ventricular lead 406 may be achieved via subclavian vein access and a preformed guiding catheter for insertion of the LV electrodes 413, 417 adjacent to the left ventricle.

Unipolar pacing and/or sensing in the left ventricle may be implemented, for example, using the LV distal electrode referenced to the can electrode 409. The LV distal electrode 413 and the LV proximal electrode 417 may be used together as bipolar sense and/or pace electrodes for the left ventricle. Pacing delivered to the heart via the left ventricular lead 406 and the right ventricular lead 404 may be used to provide cardiac resynchronization therapy such that the ventricles of the heart are paced substantially simultaneously, or in phased sequence, to provide enhanced cardiac pumping efficiency for patients suffering from chronic heart failure.

The right atrial lead 405 includes a RA-tip electrode 456 and an RA-ring electrode 454 positioned at appropriate locations in the right atrium for sensing and pacing the right atrium. In one configuration, the RA-tip 456 referenced to the can electrode 409, for example, may be used to provide unipolar pacing and/or sensing in the right atrium. In another configuration, the RA-tip electrode 456 and the RA-ring electrode 454 may be used to provide bipolar pacing and/or sensing.

Figure 4 illustrates one embodiment of a left atrial lead system 410. In this example, the left atrial lead 410 is implemented as an extracardiac lead with LA distal 418 and LA proximal 415 electrodes positioned at appropriate locations outside the heart for sensing and pacing the left atrium. Unipolar pacing and/or sensing of the left atrium may be accomplished, for example, using the LA distal electrode 418 to the can 409 pacing vector. The LA proximal 415 and LA distal 418 electrodes may be used together to implement bipolar pacing and/or sensing of the left atrium.

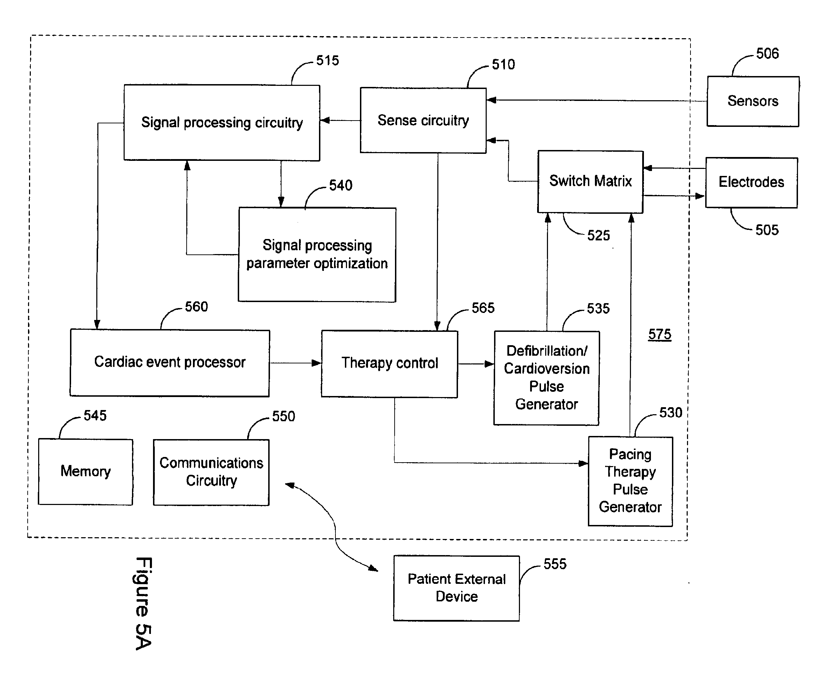

Circuitry 575 used to classify cardiac events and to control the delivery of therapy is enclosed within the housing of the device 400. Figure 5A presents a functional block diagram of CRM circuitry 575 in accordance with one embodiment. It is understood by those skilled in the art that there exist many possible configurations in which these functional blocks can be arranged. The exemplary device depicted in Figure 5A is one possible functional arrangement. Other arrangements are also possible. For example, more, fewer or different functional blocks may be used to describe a device suitable for implementing the methodologies of the present invention.

The CRM device circuitry 575 is typically powered by an electrochemical battery (not shown). A memory 545 stores data and program commands used to implement cardiac event identification according to the embodiments of the present invention as well as other operations such as therapy delivery. Data and program commands may be transferred between the device circuitry 575 and a patient-external device 555 via telemetry-based communications circuitry 550.

The circuitry 575 includes a therapy control processor 565 capable of controlling the delivery of pacing pulses and/or defibrillation shocks to the right ventricle, left ventricle, right atrium and/or left atrium. The pacing pulse generator 530 is configured to generate pacing pulses for treating bradyarrhythmia, for example, or for synchronizing the contractions of contralateral heart chambers using biatrial and/or biventricular pacing. Furthermore, under control of the therapy control processor 565, the cardioversion/defibrillation pulse generator 535 may be used to generate high energy shocks to terminate tachyarrhythmia episodes.

The pacing pulses and/or defibrillation shocks are delivered via multiple cardiac electrodes 505 disposed at various locations within, on, or about the heart and electrically coupled to the heart. In certain configurations, multiple electrodes may provide multiple sensing and/or stimulation sites within a single heart chamber. The electrodes 505 are coupled to switch matrix circuitry 525 that is used to selectively couple the electrodes 505 to the sense circuitry 510 and the therapy pulse generators 530, 535.

Cardiac electrical activity may be sensed from the electrode sites of the patient's heart are sensed via electrodes 505 and sense circuitry 510. Other types of signals, e.g., heart sound signals, pressure signals, respiration signals, and/or other types of signals may be sensed using various sensors 506 in conjunction with sense circuitry 510. Sensed signals acquired using the sensors 505, 506 and sense circuitry 510 are processed by signal processing circuitry 515 to enhance cardiac signal features used for cardiac event detection. The type of processing applied via the signal processing circuitry 515 may be based on the type of cardiac event being identified. For example, in one situation, the morphology of the cardiac signal component of the sensed signal may be altered to enhance a first set of features to facilitate detection of the cardiac pacing responses. In a second situation, the morphology of the cardiac signal component of the sensed signal may be altered to enhance a second set of features to facilitate detection of tachyarrhythmia or to identify a type of tachyarrhythmia.

Altering the morphology of the cardiac signal component of the sensed signal is accomplished by filtering the signal to remove certain frequency components. For example, high pass, low pass or band pass filtering may be applied. Additionally the cardiac signal morphology may be altered by changing data acquisition parameters such as A/D converter resolution or sample frequency. The signal acquired via the electrodes 505 or sensors 506 and sense circuitry 510 may also be filtered to remove or reduce the noise component from the sensed signal.

The cardiac signal that has been modified to enhance feature detection is analyzed by cardiac event processor 560. During this analysis, cardiac signal features present in the processed signal are used to classify various cardiac events. In one scenario, cardiac signal features present in a processed electrogram (EGM) signal are used by the cardiac event processor 560 to classify the cardiac response to pacing. For example, the presence or absence of cardiac signal peaks within cardiac response detection windows may be used to determine whether or not the pacing pulse produced capture or some other pacing response such as noncapture, fusion or pseudofusion, or noncapture with intrinsic activation.

In another implementation, the cardiac signal modified by the signal processing circuitry 515 may be used to classify cardiac tachyarrhythmia episode types. Features extracted from the processed signal of cardiac episode beats may be compared to template features that are representative of various types of tachyarrhythmia. The amplitude, timing, and/or other characteristics of the processed signal features of episode beats are compared to corresponding template features. If the episode beat features and template features are sufficiently similar, then the cardiac event processor 560 identifies the cardiac episode as the type of tachyarrhythmia represented by the template.

In some implementations, the parameters of the signal processing circuitry 515 may be selected based on population data. For example, a fixed filter configuration may be chosen to process the signal, where the fixed filter provides a desired morphology of signal features across the general patient population. In one capture application, a filter with a high pass corner frequency of about 3 Hz and a low pass corner frequency of about 100 Hz produces negative and positive signal peaks that occur with a desired amplitude and timing. Determination of the cardiac pacing response may involve sensing for the presence of positive and/or negative peaks within detection windows. For most patients, these signal processing parameters produce cardiac signals that provide accurate cardiac pacing response determination based on the location and timing of the cardiac signal peaks.

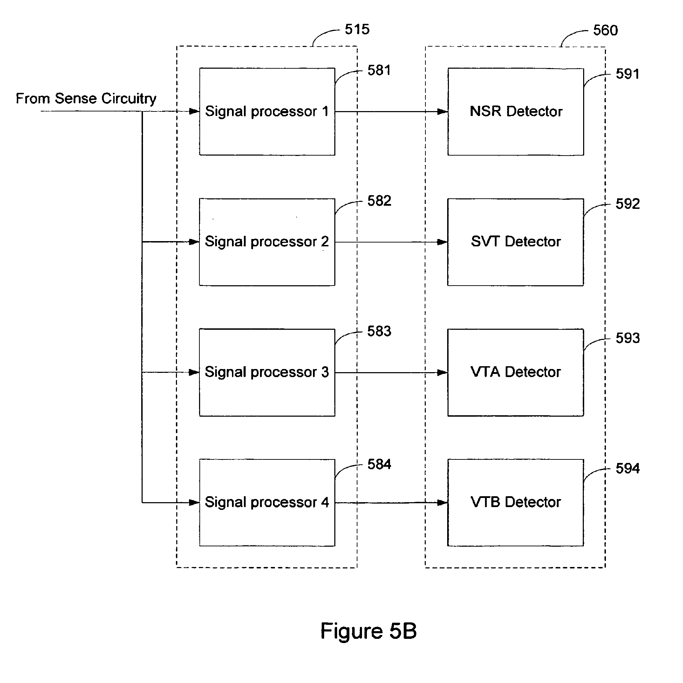

As illustrated in Figure 5B, in certain embodiments, the signal processing circuitry 515 may be implemented as a bank of signal processing units 581-584, where each signal processing unit 581-584 has its own set of parameters. For example, each signal processing unit 581-584 may use parameters selected to enhance feature detection for a particular type of cardiac event. The cardiac signal from sense circuitry 510 (Figure 5A) may be processed by each of the signal processing units 581-584. The outputs of the signal processing units 581-584 are analyzed by the cardiac event processor 560 to detect the presence of the cardiac events.

For example, as illustrated in Figure 5B, a first signal processing unit 581 may be configured to enhance feature detection for normal sinus rhythm (NSR). A second signal processing unit 582 may be configured to enhance feature detection for supraventricular tachyarrhythmia (SVT). A third signal processing unit 583 may be configured to enhance feature detection for a first type of ventricular tachyarrhythmia (VTA). A fourth signal processing unit 584 may be configured to enhance feature detection for a second type of ventricular tachyarrhythmia (VTB). The outputs of the signal processing units 582-584 are applied to the cardiac event processor 560 which may include circuitry for discrimination of NSR 591, SVT 592, VTA 593, and VTB 594. In one implementation, the cardiac event processor 560 may include circuitry 591-594 to compare the signals processed by the signal processing units 581-584 to templates representing NSR, SVT, VTA, and VTB. Identification of the cardiac events may be based on the degree of correlation between the processed cardiac signals and the templates.

Due to disease state, patient pathophysiology, sensor characteristics and/or lead implant characteristics, the uniform parameter settings described above may result in sub-optimal feature detection for certain patients. In some embodiments, the signal processing parameter settings may be programmable via an external device programmer or other remote device management system. For example, in one scenario, a physician or other health care provider may select the parameters for signal processing and observe changes in the cardiac signal processed using the selected parameters. Through observation of the cardiac signal, the physician may determine if the selected parameters produce a desired improvement in cardiac signal morphology to achieve enhanced feature detection. In another scenario, the physician may determine if the modified parameters produce a desired effect, e.g., more accurate capture detection and/or identification of tachyarrhythmia episodes.

In some embodiments, selection of the individualized parameter settings may be performed automatically by the device. For example, the device implement a process wherein the parameter settings are automatically incrementally changed and the signal produced using each of the parameter setting is analyzed. This process may continue until one or more undesirable features are reduced in the cardiac signal until desirable features are accentuated, or until optimal parameter settings are determined. For example, in one implementation, the device may identify an undesirable feature in the signal and then automatically step through the available signal processing parameter settings to determine whether, or the degree to which, each setting removes or sufficiently reduces the undesirable feature. If it is not possible to eliminate the undesirable signal feature from the cardiac signal, an optimal parameter setting which provides the greatest reduction of the undesirable feature is selected. In other implementations, desirable features may be accentuated through the use of automatic modification of the signal processing parameters. For example, the device may step through available signal processing parameter settings to determine an optimal parameter setting that produces a sharp signal peak.

Methods to accentuate desirable features, and/or to determine optimal parameter settings for signal processing may be implemented in firmware, software or a combination of both firmware and software. The automated parameter selection process may be performed on demand, periodically, and/or when degradation of cardiac event identification is detected. For example, if capture detection cannot be achieved or is intermittent, the signal processing parameter settings used for capture detection may be tested and readjusted.

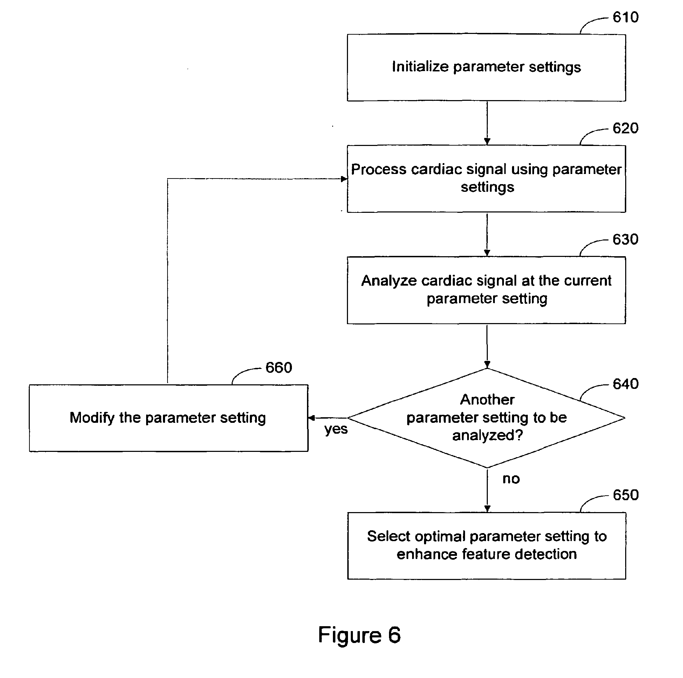

The flow graph of Figure 6 illustrates a method for automatically determining signal processing parameters for feature enhancement in accordance with embodiments of the invention. The parameter settings of the signal processing circuitry are initialized 610. The cardiac signal is sensed and processed 620 using the initial parameter settings. The processed cardiac signal is analyzed 630 with respect to the presence of undesirable features that may interfere with cardiac event identification. If there is another signal processing parameter setting to be tested 640, the parameters of the signal processing circuitry are modified 660 to the new setting. The cardiac signal is sensed and processed 620 with the new parameter setting. The processed signal is again analyzed 630 with respect to the presence of undesirable features. This process continues until all parameter settings have been tested. The optimal parameter setting for feature enhancement is selected 650 after all parameter settings have been tested.

The presence of undesirable features in the cardiac signal may be determined using various techniques. Several exemplary techniques are described herein, but any technique that detects the presence of an undesirable feature or analyzes the comparative strength of desirable and undesirable features may be used.

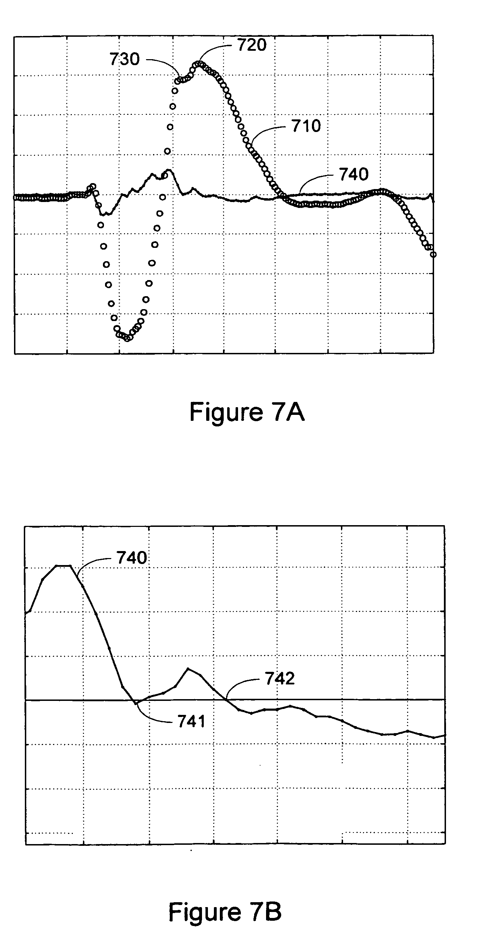

A technique using gradient analysis for determining the presence of undesirable features is described in the context of a cardiac signal used for capture detection. Figure 7A illustrates a cardiac signal 710 having a morphology exhibiting double positive peaks 720, 730. As previously discussed, capture detection may rely on a signal peak occurring within a detection window having predetermined amplitude and timing ranges. The presence of a double peak may lead to uncertainties in capture detection, for example, when one peak falls within the window and one peak falls outside the window. Therefore it is desirable to eliminate the double peak by appropriate signal processing.

In accordance with one embodiment, the presence of a multiple peaks in a cardiac signal may be detected using a gradient analysis. In this process, the gradient 740 of the cardiac signal 710 is determined. The cardiac signal gradient 740 is graphically illustrated in Figures 7A and 7B. Peaks in the cardiac signal 710 are indicated in the gradient by zero crossings. The main peak 720 of the cardiac signal 710 is indicated in the gradient 740 by zero crossing 742. The additional peak 730 in the vicinity of the main peak 720 is indicated in the gradient 740 by zero crossing 741.

If one or more additional zero crossings are detected in the cardiac signal gradient 740 at locations close to the location of the main peak zero crossing, as illustrated in Figures 7A and 7B, the signal processing parameters for this particular cardiac signal morphology are non-optimal. The signal processing parameters may be changed to eliminate or reduce the presence of the secondary peaks.

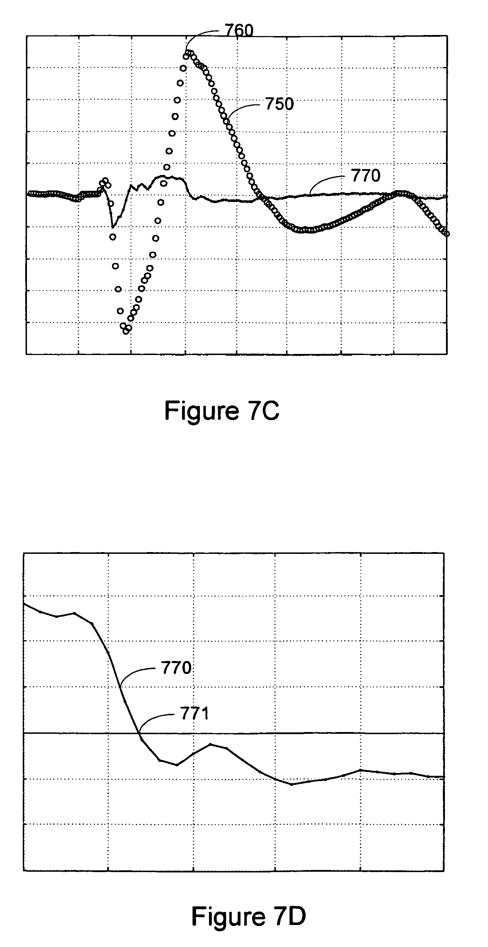

Figures 7C and 7D illustrate the cardiac signal 750 after it has been processed to reduce the double peak. The processed cardiac signal 750 exhibits a single peak 760 that is indicated in the cardiac signal gradient 770 by zero crossing 771.

Another method for detecting undesirable features in the cardiac signal such as the multiple peaks illustrated in Figure 7A involves the use of a curvature-based analysis. Through curvature analysis significant points of the cardiac signal are determined based on the curvature of the cardiac signal, along with peak detection. If additional significant points indicating secondary peaks having amplitude and location in the vicinity of the main peak, then the signal processing parameter settings may be modified to eliminate the secondary peaks. The use of curvature analysis to detect cardiac signal features including peaks and/or inflection points, for example, is further described in commonly owned U.S. Patent 6,950,702 .

Modification of cardiac signal processing parameters to enhance feature detection in accordance with embodiments of the invention may be applied to improve discrimination of tachyarrhythmia events. In morphology based arrhythmia detection, feature points of cardiac signal are extracted, and the correlation with features from a template is calculated. If the correlation coefficient is higher than a predetermined threshold, the beat is classified as the type of beat represented by the template.

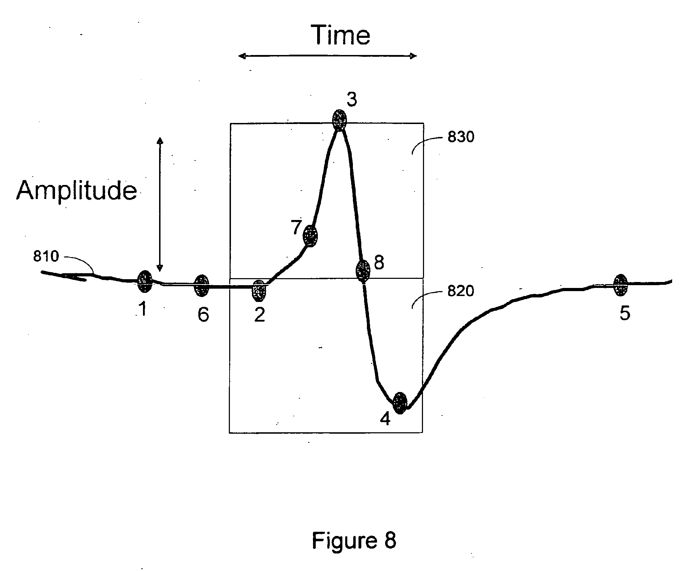

In one implementation, the detected beat signal is compared to a template representative of supraventricular rhythm (SVR). If the beat signal is correlated to the SVR template, the beat is classified as SVR and therapy is withheld. If the beat signal is not correlated to the SVR template, the beat is classified as a ventricular tachyarrhythmia beat and therapy may be delivered. According to this approach, feature points 1-8 are extracted from the cardiac signal 810, as illustrated in Figure 8. The use of eight feature points is based on the assumption that only 2 inflection points (feature 2 and feature 4 in Figure 8) exist in a region 820 around the peak feature 3 for a SVR beat. If there are additional inflection points in region 820, features 2, 3 and 4 may not be identified correctly during template formation due to variation in determining these feature points. The rhythm analysis is further based on the assumption that there is no significant variation in curvature in region 830. If this is not the case, the local changes in morphology may cause variation in determining features 2, 3, 4, 7 and 8 during beat to beat correlation as well as during template formation. The feature point variation caused by local changes in morphology may impact the ability to accurately classify the type of cardiac rhythm.

In accordance with the approaches of the present invention, signal processing parameters may be determined that eliminate the undesirable morphology or curvature that occur in regions 820 and 830. The above-described gradient or curvature based methods may be used to detect the undesirable inflection points. For example, inflection points may be detected using the gradient approach as points where the gradient is zero.

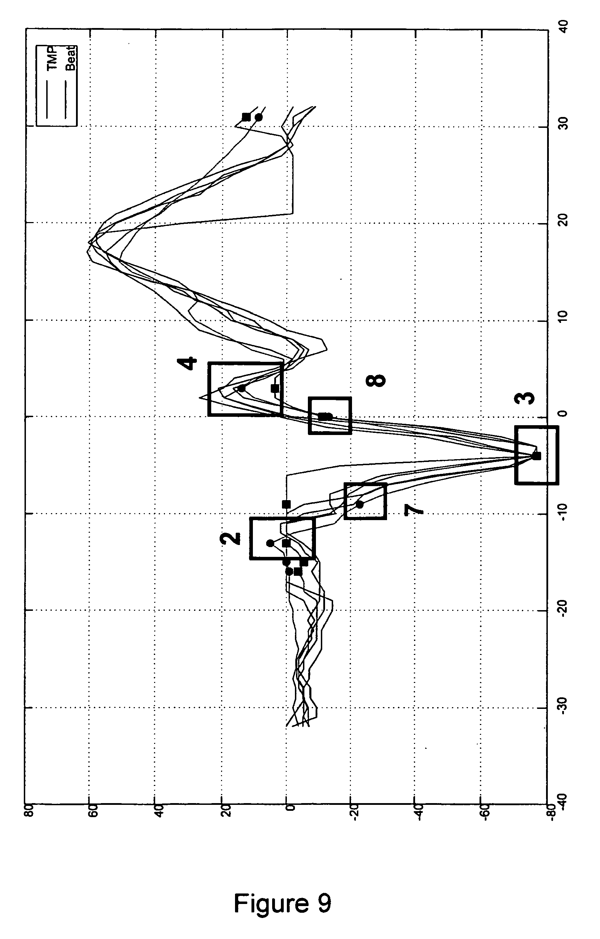

Figure 9 illustrates the windows drawn around the QRS complex feature point locations (features 2, 3, 4, 7 and 8) which serve to facilitate detection of significant changes in curvature and/or the presence of multiple inflection points within the window area. The signal processing parameters may then be selected to reduce or eliminate the undesirable features in the window area. The signal processing parameters may be optimized to produce the best correlation between beats from stored episodes and a template beat. If the signal processing parameters are significantly different after the adjustment has been made, a new template update may be initiated and the resulting template compared to the previous template stored in memory. If both templates are morphologically different, the new template may be stored in memory, replacing the old template.

There are also other approaches to detect undesirable features or enhance desirable features in the cardiac signal component, such as using energy or power of the signal. For example, for the capture detection example, it may be possible to integrate the positive and negative deflections and choose the signal processing parameters which produce the smallest integral as the optimal parameter.

Determination of the signal processing parameters may be performed based on patient population data or may be individualized for a particular patient. As previously discussed, the signal processing parameters may be automatically or manually determined. In one approach, the signal processing parameters may be determined during template creation and/or update. In another approach, the signal processing parameters may be determined based on the patient's stored episode data. In another approach, the signal processing parameters may be selected by a physician and uploaded to the device. In yet another approach, the physician could run a pacing or exercise protocol to simulate tachyarrhythmia with the device adjusting the signal processing parameters during the simulated tachyarrhythmia to enhance tachyarrhythmia detection.

Various modifications and additions can be made to the embodiments discussed hereinabove without departing from the scope of the present invention. Accordingly, the scope of the present invention should not be limited by the particular embodiments described above, but should be defined only by the claims set forth below and equivalents thereof.

## Claims

1. A method for classifying a cardiac event, comprising:
sensing (110) a signal (210, 305) comprising a cardiac signal component and a noise signal component;
processing (120) the sensed signal by filtering to alter morphology of one or more features (230, 312) of the cardiac signal component to accentuate the one or more features (250) of the cardiac signal component of the sensed signal, the altered morphology of the cardiac signal component enhancing detection of the one or more features of the cardiac signal component;
detecting (130) the one or more features; and
classifying (140) the cardiac event using the one or more detected features.

2. The method of claim 1, further comprising automatically determining signal processing parameters used to alter morphology of the one or more features of the cardiac signal component of the sensed signal.

3. The method of claim 2, wherein determining the signal processing parameters comprises determining the signal processing parameters that enhance a correlation coefficient between beats from cardiac episodes and a template.

4. The method of claim 2, wherein determining the signal processing parameters comprises determining the signal processing parameters based on an energy or power of the cardiac signal component.

5. The method of claim 1, wherein the sensed cardiac component comprises a peak (312) that falls outside a detection window (310) and processing the sensed cardiac signal alters the peak (311) to fall within the detection window.

6. A medical system (400), comprising:
sensing circuitry (510) configured to sense a signal comprising a cardiac signal component and a noise component;
signal processing circuitry (515, 540) configured to process the sensed signal by filtering to alter morphology of one or more features (230, 312) of the cardiac signal component to accentuate the one or more features (250) of the cardiac signal component of the sensed signal, the altered cardiac signal component enhancing detection of the one or more features of the cardiac signal component;
a feature detector (560) configured to detect the one or more features of the cardiac signal component; and
a cardiac event processor (560) configured to classify a cardiac event using the one or more detected features.

7. The system of claim 6, further comprising a noise filter (515) configured to filter the sensed signal to reduce the noise component of the sensed signal.

8. The system of claim 6, wherein parameters of the signal processing circuitry (515, 540) are derived from population based data.

9. The system of claim 6, wherein parameters of the signal processing circuitry (515, 540) are determined based on individual characteristics of a patient.

10. The system of claim 6, wherein parameters of the signal processing circuitry (515, 540) are automatically adaptable.

11. The system of claim 6, wherein the cardiac event processor (560) comprises circuitry configured to determine a cardiac pacing response.

12. The system of claim 6, wherein the signal processing circuitry (515, 540) is configured to alter morphology of the one or more features of the cardiac signal (710, 750) component by one or both of reducing a double peak feature (720, 730) to a single peak feature (760) and producing a sharp signal peak feature.

13. The system of claim 6, wherein the signal processing circuitry (515, 540) comprises a bank of signal processing units (581-584), wherein a first signal processing unit comprises circuitry configured to enhance detection of a first type of cardiac event and a second signal processing unit comprises circuitry configured to enhance detection of a second type of cardiac event.

14. The system of claim 13, wherein the first type of cardiac event comprises normal sinus rhythm and the second type of cardiac event comprises supraventricular tachyarrhythmia.

15. The system of claim 13, wherein the first type of cardiac event comprises a first type of ventricular tachyarrhythmia and the second type of cardiac event comprises a second type of ventricular tachyarrhythmia.

## Patentansprüche

1. Verfahren zum Klassifizieren eines Herzvorfalles mit:
Erfassen (110) eines Signals (210, 305) mit einer Herzsignalkomponente und einer Rauschsignalkomponente;
Verarbeiten (120) des erfassten Signals durch Filtern zum Verändern der Morphologie von einem oder mehreren Merkmalen (230, 312) der Herzsignalkomponente zum Betonen des einen oder der mehreren Merkmale (250) der Herzsignalkomponente des erfassten Signals, wobei die geänderte Morphologie der Herzsignalkomponente die Erfassung des einen oder der mehreren Merkmale der Herzsignalkomponente verstärkt;
Erfassen (130) des einen oder der mehreren Merkmale; und
Klassifizieren (140) des Herzvorfalls unter Verwendung des einen oder der mehreren erfassten Merkmale.

2. Verfahren nach Anspruch 1, des Weiteren mit einem automatischen Bestimmen von Signalverarbeitungsparametern, die zum Verändern der Morphologie des einen oder der mehreren Merkmale der Herzsignalkomponente des erfassten Signals verwendet werden.

3. Verfahren nach Anspruch 2, bei dem das Bestimmen der Signalverarbeitungsparameter ein Bestimmen der Signalverarbeitungsparameter umfasst, die einen Korrelationskoeffizienten zwischen Schlägen von Herzepisoden und einer Vorlage verstärken.

4. Verfahren nach Anspruch 2, bei dem das Bestimmen der Signalverarbeitungsparameter ein Bestimmen der Signalverarbeitungsparameter beruhend auf einer Energie oder Leistung der Herzsignalkomponente umfasst.

5. Verfahren nach Anspruch 1, bei dem die erfassten Herzkomponente einen Peak (312), der aus einem Erfassungsfenster (310) herausfällt, umfasst, und wobei die Verarbeitung des erfassten Herzsignals den Peak (311) ändert, so dass er in das Erfassungsfenster fällt.

6. Medizinisches System (400) mit:
einer Erfassungsschaltung (510), die ausgestaltet ist, um ein Signal zu erfassen, das ein Herzsignalkomponente und eine Rauschkomponente umfasst;
einer Signalverarbeitungsschaltung (515, 540), die ausgestaltet ist, um das erfasste Signal zu verarbeiten, in dem zur Änderung der Morphologie eines oder mehrerer Merkmale (230, 312) die Herzsignalkomponente gefiltert wird, um das eine oder die mehreren Merkmale (250) der Herzsignalkomponente des erfassten Signals zu betonen, wobei die geänderte Herzsignalkomponente die Erfassung des einen oder mehreren Merkmale der Herzsignalkomponente verstärkt,
einem Merkmalsdetektor (560), der ausgestaltet ist, um das eine oder die mehreren Merkmale der Herzsignalkomponente zu erfassen; und
einem Herzvorfallprozessor (560), der ausgestaltet ist, um einen Herzvorfall unter Verwendung des einen oder der mehreren Merkmale zu klassifizieren.

7. System nach Anspruch 6, des Weiteren mit einem Rauschfilter (515), das ausgestaltet ist, um das erfasste Signal zu filtern, um die Rauschkomponente des erfassten Signals zu verringern.

8. System nach Anspruch 6, bei dem die Parameter der Signalverarbeitungsschaltung (515, 540) auf bevölkerungsbasierten Daten ermittelt werden.

9. System nach Anspruch 6, bei dem die Parameter der Signalverarbeitungsschaltung (515, 540) beruhend auf individuellen Charakteristiken eines Patienten bestimmt werden.

10. System nach Anspruch 6, bei dem die Parameter der Signalverarbeitungsschaltung (515, 540) automatisch anpassbar sind.

11. System nach Anspruch 6, bei dem der Herzvorfallprozessor (560) eine Schaltung umfasst, die ausgestaltet ist, um eine Herz-Pacing-Antwort zu bestimmen.

12. System nach Anspruch 6, bei dem die Signalverarbeitungsschaltung (515, 540) ausgestaltet ist, um die Morphologie von einem oder mehreren Merkmalen der Herzsignalkomponente (710, 750) zu ändern, indem ein DoppelPeak-Merkmal (720, 730) auf ein Einzel-Peak-Merkmal (760) verringert wird und/oder ein scharfes Einzel-Peak-Merkmal erzeugt wird.

13. System nach Anspruch 6, bei dem die Signalverarbeitungsschaltung (515, 540) eine Bank aus Signalverarbeitungseinheiten (581-584) umfasst, wobei eine erste Signalverarbeitungseinheit eine Schaltung umfasst, die ausgestaltet ist, um die Detektion eines ersten Typs von Herzvorfall zu erfassen, und eine zweite
Signalverarbeitungseinheit eine Schaltung umfasst, die ausgestaltet ist, um einen zweiten Typ von Herzvorfall zu erfassen.

14. System nach Anspruch 13, bei dem der erste Typ von Herzvorfall einen normal Sinusrhythmus und der zweite Typ von Herzvorfall supraventrikulare Tachyarrhythmie umfasst.

15. System nach Anspruch 13, bei dem der erste Typ von Herzvorfall einen ersten Typ von ventrikularer Tachyarrhythmie und der zweite Typ von Herzvorfall einen zweiten Typ von ventrikularer Tachyarrhythmie umfasst.

## Revendications

1. Procédé de classification d'un événement cardiaque, comprenant :
la détection (110) d'un signal (210, 305) comprenant une composante de signal cardiaque et une composante de signal de bruit ;
le traitement (120) du signal détecté par filtrage pour modifier la morphologie d'une ou plusieurs particularités (230, 312) de la composante de signal cardiaque pour accentuer les une ou plusieurs particularités (250) de la composante de signal cardiaque du signal détecté, la morphologie modifiée de la composante de signal cardiaque renforçant la détection des une ou plusieurs particularités de la composante de signal cardiaque ;
la détection (130) des une ou plusieurs particularités ; et
la classification (140) de l'événement cardiaque en utilisant une ou plusieurs particularités détectées.

2. Procédé selon la revendication 1, comprenant en outre la détermination automatique de paramètres de traitement de signal utilisés pour modifier une morphologie des une ou plusieurs particularités de la composante de signal cardiaque du signal détecté.

3. Procédé selon la revendication 2, dans lequel la détermination des paramètres de traitement de signal comprend la détermination des paramètres de traitement de signal qui renforcent un coefficient de corrélation entre des battements issus d'épisodes cardiaques et un modèle.

4. Procédé selon la revendication 2, dans lequel la détermination des paramètres de traitement de signal comprend la détermination des paramètres de traitement de signal d'après une énergie ou une puissance de la composante de signal cardiaque.

5. Procédé selon la revendication 1, dans lequel la composante cardiaque détectée comprend un pic (312) qui tombe à l'extérieur d'une fenêtre de détection (310) et le traitement du signal cardiaque détecté modifie le pic (311) pour qu'il tombe dans la fenêtre de détection.

6. Système médical (400), comprenant :
une circuiterie de détection (510) configurée pour détecter un signal comprenant une composante de signal cardiaque et une composante de bruit ;
une circuiterie de traitement de signal (515, 540) configurée pour traiter le signal détecté par filtrage pour modifier la morphologie d'une ou plusieurs particularités (230, 312) de la composante de signal cardiaque pour accentuer les une ou plusieurs particularités (250) de la composante de signal cardiaque du signal détecté, la composante de signal cardiaque modifiée renforçant la détection des une ou plusieurs particularités de la composante de signal cardiaque,
un détecteur de particularités (560) configuré pour détecter les une ou plusieurs particularités de la composante de signal cardiaque ; et
un processeur d'événement cardiaque (560) configuré pour classifier un événement cardiaque en utilisant les une ou plusieurs particularités détectées.

7. Système selon la revendication 6, comprenant en outre un filtre de bruit (515) configuré pour filtrer le signal détecté pour réduire la composante de bruit du signal détecté.

8. Système selon la revendication 6, dans lequel des paramètres de la circuiterie de traitement de signal (515, 540) sont dérivés de données basées sur une population.

9. Système selon la revendication 6, dans lequel des paramètres de la circuiterie de traitement de signal (515, 540) sont déterminés d'après des caractéristiques individuelles d'un patient.

10. Système selon la revendication 6, dans lequel des paramètres de la circuiterie de traitement de signal (515, 540) sont automatiquement adaptables.

11. Système selon la revendication 6, dans lequel le processeur d'événement cardiaque (560) comprend une circuiterie configurée pour déterminer une réponse de stimulation cardiaque.

12. Système selon la revendication 6, dans lequel la circuiterie de traitement de signal (515, 540) est configurée pour modifier la morphologie des une ou plusieurs caractéristiques de la composante de signal cardiaque (710, 750) par un ou deux éléments parmi la réduction d'une particularité de pic double (720, 730) en une particularité de pic simple (760) et la production d'une particularité de pic de signal net.

13. Système selon la revendication 6, dans lequel la circuiterie de traitement de signal (515, 540) comprend un banc d'unités de traitement de signal (581 à 584), dans lequel une première unité de traitement de signal comprend une circuiterie configurée pour renforcer la détection d'un premier type d'événement cardiaque et une seconde unité de traitement de signal comprenant une circuiterie configurée pour renforcer la détection d'un second type d'événement cardiaque.

14. Système selon la revendication 13, dans lequel le premier type d'événement cardiaque comprend un rythme sinusal normal et le second type d'événement cardiaque comprend une tachyarythmie supraventriculaire.

15. Système selon la revendication 13, dans lequel le premier type d'événement cardiaque comprend un premier type de tachyarythmie ventriculaire et le second type d'événement cardiaque comprend un second type de tachyarythmie ventriculaire.
